(19) Europäisches Patentamt
European Patent Office
Office européen des brevets

(11) Publication number: 0 189 938 A2

(12) # EUROPEAN PATENT APPLICATION

(21) Application number: 86101297.9

(22) Date of filing: 31.01.86

(51) Int. Cl.⁴: C 12 P 13/22
C 12 N 9/10, C 12 N 15/00

(30) Priority: 01.02.85 US 697184

(43) Date of publication of application: 06.08.86 Bulletin 86/32

(84) Designated Contracting States: DE FR GB IT

(71) Applicant: G.D. Searle & Co.
P.O. Box 1045
Skokie Illinois 60076(US)

(72) Inventor: Dyer, Robert Laurence
10 Tilbury Wood Close
Downley High Wycombe(GB)

(72) Inventor: Lewis, David John
Kiln Road
Prestwood(GB)

(74) Representative: Wolff, Hans Joachim, Dr.jur. Dipl.-Chem. et al,
Beil, Wolff & Beil Rechtsanwälte Postfach 80 01 40
Adelonstrasse 58
D-6230 Frankfurt am Main 80(DE)

(54) **Process for the preparation of L-DOPA.**

(57) This disclosure relates to a process for preparing exclusively the L-isomer of 3, 4-dihydroxyphenylalanine. The process comprises transaminating a blocked keto acid of the formula

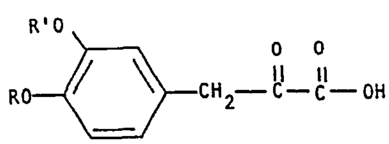

wherein R and $R^1$ are independently hydrogen or $C_1$-$C_4$ alkyl provided that R and $R^1$ are not both hydrogen; to yield the corresponding blocked amino acid. The blocked amino acid is deprotected to yield exclusively the L-isomer of 3, 4-dihydroxyphenylalanine.

EP 0 189 938 A2

0189938

Our No. 24 973

G.D. Searle & Co.
Skokie, Il. 60076, U.S.A.

# PROCESS FOR THE PREPARATION OF L-DOPA

## BACKGROUND OF THE INVENTION

L-DOPA (3,4-dihydroxyphenylalanine) is an effective therapeutic agent in the treatment of Parkinson's disease. Numerous methods for preparing L-DOPA are known. For example, USP 3,714,242, USP 4,005,127 and USP 2,605,282, describe various synthetic routes for the preparation of L-DOPA. However, all of the known processes for preparing L-DOPA yield a mixture of the optical isomers of 3,4-dihydroxyphenylalanine, thus requiring additional steps to separate the desired L-isomer from the other isomers produced. It is the object of the present invention to provide a procedure for preparing exclusively the L-isomer of 3,4-dihydroxyphenylalanine.

SUMMARY OF THE INVENTION

The present invention related to a process for preparing L-3,4-dihydroxyphenylalanine comprising:

a) reacting a blocked keto acid of the formula:

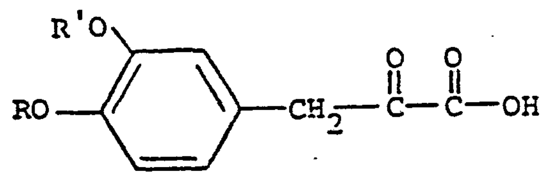

(I)

wherein R and R' are independently hydrogen or $C_1$-$C_4$ alkyl provided that R and $R^1$ are not both hydrogen; with an amine donor in the presence of an aminotransferase obtained from a microorganism which has been genetically modified to overproduce the aminotransferase to yield a blocked amino acid of the formula:

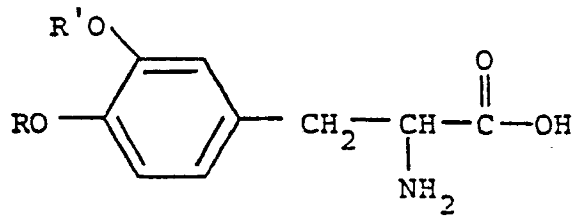

(II)

b) deprotecting the blocked amino acid to yield the L-3,4-dihydroxyphenylalanine.

The process at the present invention is particularly useful in that the process yields only the L-isomer of 3,4-dihydroxyphenylalanine.

## DETAILED DESCRIPTION OF THE INVENTION

Synthesis of L-DOPA, in accordance with the present invention, may proceed in accordance with the following reaction mechanism:

STEP A:

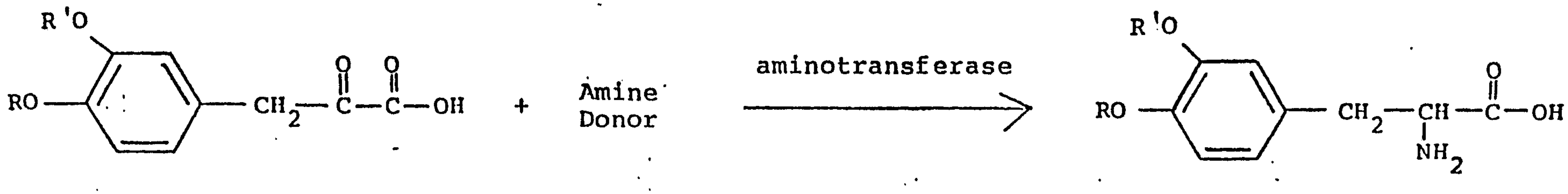


STEP B:

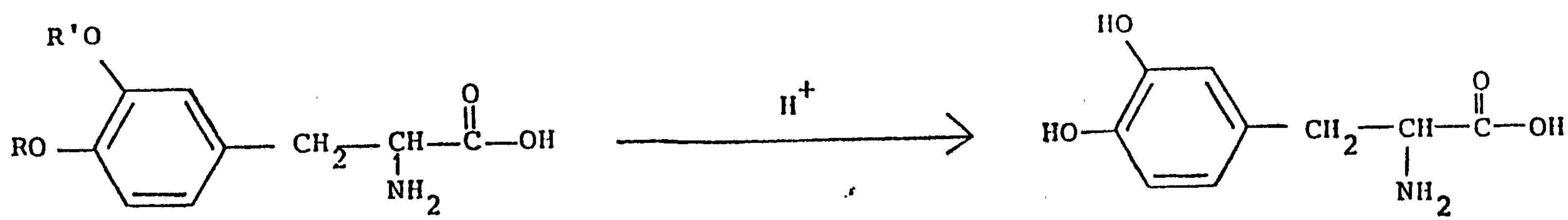

The blocked keto acid of formula (I) may be prepared in accordance with numerous synthetic routes. In a preferred embodiment a substituted benzaldehyde of the formula

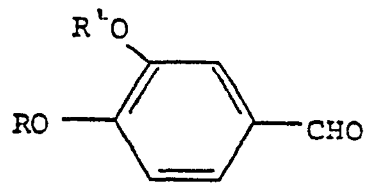

(III)

wherein R and R' are independently hydrogen or $C_1$-$C_4$ alkyl provided that and R and R' both are not hydrogen; is heated with hydantoin in the presence of an organic base in water at a temperature greater than 50°C and preferably from about 80°C to 100°C to yield a substituted 5- benzylidene hydantoin of the formula

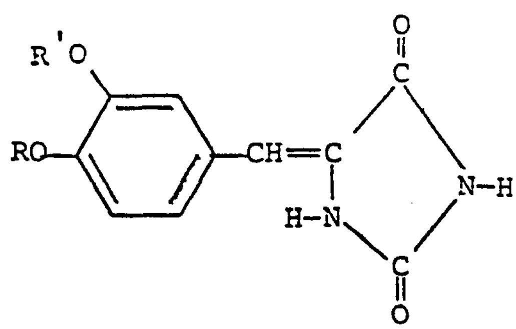

(IV)

The organic base catalyzes the reaction of the substituted benzaldehyde of formula (III) and hydantoin. The specific organic base is readily ascertained by one of ordinary skill in the art. Representative organic bases include, for example

alkanolamines, pyridines, piperidines and the like. It is preferred to employ an alkanolamine, in particular ethanolamine. The ratio of substituted benzaldehyde hydantoin is not critical. Although a 1:1 molar ratio may be employed, in practice it is preferred to employ an excess of hydantoin. It is necessary to employ a sufficient amount of organic base to catalyze the reaction, and such amounts are readily ascertained by one of ordinary skill in the art.

The substituted 5-benzylidene hydantoin of formula (IV) is hydrolyzed using a base at reflux temperatures to yield a salt of the blocked keto acid of formula (I). The specific base employed to hydrolyze the substituted 5-benzylidene hydantoin of formula (IV) may be readily ascertained by one of ordinary skill in the art. Representative of such bases include for example alkaline hydroxides such as sodium hydroxide and potassium hydroxide and the like. Although, a molar ratio of 1:1 of substituted 5- benzylidene hydantoin : base may be employed, it is preferred to employ an excess of base and most preferably a 1:10 ratio of substituted 5-benzylidene hydantoin : base. The solution containing the salt of the blocked keto acid is, after addition of the amino donor, adjusted to the pH at which the transamination reaction is conducted using a strong acid such as concentrated sulfuric acid.

The blocked keto acid of formula (I) is reacted with an amine donor in the presence of an aminotransferase obtained from a microorganism that has been genetically modified to

overproduce the aminotransferase, to yield a blocked amino acid of formula (II).

In accordance with the transamination reaction (step A) high yields of the blocked amino acid are obtained at a high rate of production utilizing a transamination reaction employing a genetically modified microorganism that overproduces the aminotransferase. The term "genetically modified microorganism that overproduces the aminotransferase" refers to a genetically modified microorganism that produces the aminotransferase at a level of at least 200U/g dry weight cells.

In accordance with a preferred emobidment of the present invention a microorganism capable of overproducing aminotransferase to be employed in the transamination reaction is grown in suitable media. The microorganism generally contains a plasmid carrying a gene encoding an aminotransferase capable of transaminating the blocked keto acid. Such media are well recognized by one of ordinary skill in the art and include for example, a media comprising yeast extract, glutamate and mineral salts. A preferred media comprises glucose and mineral salts. Such media are generally commercially available or readily prepared by one of ordinary skill in the art. The cells are cultivated within a temperature range of from 25° to 40°C and preferably about 37°C for approximately sixteen hours or until the concentration of cells is preferably at least 10g/L and most preferably 30g/L.

The cells may be utilized in the presence of the growth media or harvested by centrifugation. In addition the cells may be utilized intact or may be treated to release the aminotransferase. Techniques for releasing the aminotransferase are well known in the art and include, for example, treatment of the cell with a surfactant, e.g., 0.2% Triton X-100 0.2% deoxycholate etc.; physical breakage via freeze/thawing; sonication; pressure disruption and the like. If the transamination reaction step of the present invention is conducted at a temperature equal to or greater than 40°C, intact cells may be utilized. In a preferred embodiment of the present invention the cells are not permeabilized prior to conducting the transamination reaction.

Therefore intact cells may be added to a mixture containing the amine donor and blocked keto acid. If the cells are permeabilized prior to conducting the transamination, a portion of the solution containing the released aminotransferase is added to a mixture containing an amine donor and blocked keto acid. Preferably the resulting reaction mixture is incubated for a period of time sufficient to allow the reaction mixture to reach equilibrium.

The transamination reaction (step A) may be conducted at a temperature greater than 25°C and preferably equal to or greater than 40°C. The transamination reaction is most preferably conducted from about 40°C to 70°C and most preferably from about 45°C to 60°C.

Depending on the specific aminotransferase employed, the pH of the reaction-mixture is maintained within a range of from 5 to 9. In accordance with the present invention, the reaction mixture is preferably maintained within a range of from 6.5 to 9 and more preferably of from 7.5 to 8.5. Suitable buffers may be used to maintain the pH of the reaction mixture within a range sufficient to maintain aminotransferase activity.

In order to increase the yield of the blocked amino acid and stabilize the transamination reaction mixture, a cofactor to the aminotransferase such as pyridoxal-5-phosphate, pyridoxine, pyridoxamine or derivatives thereof may be added to the reaction mixture. The quantity of cofactor to be added to the reaction mixture is readily ascertained by one of ordinary skill in the art.

Furthermore, the addition of a formic acid solution to the reaction mixture may increase the rate of reaction and production of the blocked amino acid. It is preferred to employ a solution having a formic acid concentration in the range of from about 0.5M to about 15M. The addition of formic acid to the reaction mixture may increase the rate of reaction by increasing the activity of the aminotransferase and stabilizing the aminotransferase particularly if the transamination reaction is conducted at temperatures equal to or greater than 40°C.

The selection of the specific amine donors employed in the process of the present invention is readily ascertained by one of ordinary skill in the art. A wide variety of amine donors may be employed depending upon the aminotransferase employed. If aspartate aminotransaminase is employed as the aminotransferase, glutamic acid, alanine, aspartic acid, tyrosine or derivatives thereof, may be employed as amine donors. Preferably glutamic acid or aspartic acid and most preferably aspartic acid is employed as the amine donor.

The preferred blocked keto acid is a compound of formula (I) wherein R is hydrogen. A more preferred blocked keto acid is a compound of formula (I) wherein R is hydrogen and R' is methyl. The blocked keto acid may be utilized in the form of the free acid or salt thereof.

The ratio of amine donor to blocked keto acid may be readily ascertained by one of ordinary skill in the art. Although, the theoretical ratio of amine donor to blocked keto acid in the transamination reaction is 1:1, it is preferred to employ an excess of the amine donor. For example, if glutamic acid is employed as the amine donor, a ratio of glutamic acid: blocked keto acid in the range of from about 3:1 to 4:1 is preferred. Furthermore, if aspartic acid is employed as the amine donor, it is preferred to employ a ratio of aspartic acid: blocked keto acid in the range of from about 1:1 to 1.5:1 and most preferred in the range of 1:1 to 1.2:1.

Concentrations of blocked keto acid greater than 10g/L are effective in the process of the present invention. Concentrations of blocked keto acid in a range of 30-60g/L are preferred. Furthermore, the blocked keto acid may be prepared in situ and employed without isolation or purification in the transamination reaction step of the present invention. The reaction by-products produced in the preparation of the blocked keto acid (ie., salts, dimers, etc.) do not affect the yields of the blocked amino acid.

In addition to temperature at which the transamination reaction is conducted and concentration of the blocked keto acid, the aminotransferase activity of the transamination mixture depends upon the amount of cells containing the aminotransferase that is added to the reaction mixture. The lower the aminotransferase activity of each individual cell, the more cells that must be added to the reaction mixture to sustain the reaction. However, the greater the amount of cells added to the reaction mixture, the greater the difficulty in isolating the amino acid product. One advantage associated with the process of the present invention in employing a genetically modified microorganism capable of overproducing the aminotransferase in lieu of a wild type microorganism is that it is possible to maximize the aminotransferase activity in the reaction mixture and minimize the total amount of cells present, thereby facilitating product isolation. The low cell density of the reaction mixture in the process of the present invention enhances recovery of the blocked amino acid product

by facilitating the removal of cell debris. A concentration of cells sufficient to yield an aminotransferase activity of 0.5 x $10^4$ U/L or greater is preferred when conducting the process of the present invention.

The blocked amino acid produced in accordance with the methods of the present invention may be isolated in accordance with conventional techniques. For example, a water miscible solvent wherein the blocked amino acid is insoluble may be added to the transamination reaction mixture. Upon addition of the water miscible solvent, the blocked amino acid will precipitate and thus be removed from the transamination reaction mixture. In addition, the blocked amino acid may be hydrolyzed to L-3,4-dihydroxyphenylalanine without isolation from the transamination reaction mixture.

In accordance with the process of the present invention, a wide variety of aminotransferases produced by a microorganism may be effective in the process of the present invention. Examples of such aminotransferases include but are not limited to aminotransferases such as aspartate aminotransferases, branched chain aminotransferases and aromatic aminotransferases as defined by the gene products E.coli genes aspC, tyrB and ilvE and the like. The above-mentioned aminotransferases when produced using a genetically modified microorganism are not necessarily pure. However, it has been found that unpurified crude extracts containing the aminotransferase of interest may be employed in the process of the present invention. These

extracts may be of course, partially purified prior to being utilized in accordance with the methods of the present invention. A mixture of two or more aminotransferases may also be employed in accordance with the methods of the present invention. Aspartate aminotransferase is the preferred aminotransferase employed.

In general, production of amino acids by conventional transamination reactions is limited by aminotransferase activity and that increasing the temperature of the reaction or keto acid concentration substantially reduces and/or effectively inhibits aminotransferase activity. Therefore in order to produce the blocked amino acid in accordance with a transamination reaction at temperatures equal to or greater than 40°C or at high keto acid concentrations , it is necessary to increase the aminotransferase activity to a level sufficiently high to overcome effects of denaturation of the aminotransferase due to temperature and/or high keto acid concentration. Because a wild type microorganism will not produce aminotransferases in quantities sufficient to conduct the processes of the present invention, it is necessary to employ a genetically modified microorganism which will over-produce the aminotransferase. As readily ascertained by one of ordinary skill in the art, microorganisms may be readily genetically modified in accordance with conventional techniques. Such techniques include, for example, mutagenesis, transduction, transformation, (e.g. with multi copy plasmids), conjugation and <u>in vitro</u> gene manipulation. For example,

aminotransferase over-producing strains may be isolated after mutagenesis. Alternatively, the gene for the appropriate aminotransferase may be cloned into a suitable plasmid or phage vector. A further embodiment invokes isolation of the aminotransferase gene on a suitable vector and the subsequent in vitro or in vivo manipulation so as to elevate expression of the aminotransferase gene. This may be accomplished for example, by fusing the gene to a more powerful constitutive promoter. Then, the isolated, mobilized aminotransferase gene may be inserted back into the microorganism chromosome by techniques such as recombination, transposition or phage interactions.

The microorganisms which may be genetically modified as described herein include but are not limited to, for example, Achromobacter aquatilis, Achromobacter liquidum, Aspergillus oryzae, Aspergillus niger, Bacillus species such as Bacillus megaterium, Bacillus subtilis, Bacterium succinium, Brevibacterium species such as Brevibacterium flavum, coryneforms including Corynebacterium species such as Corynebacterium glutaminicum, Erwinia herbicola, Escherichia coli, Gluconobacter melanogenus, Lactobacillus bulgaris, Micrococcus ureae, Penicillium vinaceum, Proteus vulgaris, Pseudomonas species such as Pseudomonas aeruginosa, Pseudomonas dacunhae, Pseudomonas putida, Sarcina lutea, Streptomyces griseus, Serratia marcescens, Streptomyces phaeochromogenus, and Saccharomyces species and Schizosaccharomyces species. These microorganisms may not

necessarily be intact living cells, but may be lyophilized, heat-treated or treated with acetone and the like to permeabilize the cells prior to use hereof in the present invention.

For the purpose of illustrating the process of the present invention, a strain rich in aminotransferase may be constructed by cloning the gene for the aspartate aminotransferase from E.coli K12 (aspC) into the multi-copy plasmid pAT153. The cloned strain is transformed into a suitable strain such as E.coli K12 W3110, and the resulting strain over-produces the aspC gene product from 10-100 times. The aspartate aminotransferase is known to be active in the transaminations; i.e., the conversion of phenylpyruvic acid to phenylalanine; using both L-aspartic acid and L-glutamic acid as amine donors. The aspC encoded protein has the additional benefit of being relatively heat stable.

An aspC clone may preferably be obtained by ligating suitably restricted DNA from either E.coli K12 or another suitable microorganism into a suitable vector such as pAT153. The resulting ligation reaction is then used to transform a suitable recipient such as HW159 (ATCC 39260) deposited in American Type Culture Collection, 12301 Parklawn Drive, Rockville, Maryland 20852. This strain (HW159) lacks both the aspartate aminotransferase (aspC) and aromatic aminotransferase (tyrB) genes. The result of these two lesions is that the strain requires tyrosine, aspartate and to

a lesser extent, phenylalanine for growth. Recombinants that carry aspC or tyrB clones may be readily identified by their ability to grow on minimal medium in the absence of tyrosine, aspartate and phenylalanine. AspC clones may be differentiated from tyrB clones by analysis of cells extracts by native polyacrylamide gel electrophoresis followed by staining for phenylpyruvic acid aminotransferase activity. Isolation of an aspC clone may be further facilitated by the use of a specialised transducing phage such as Lambda aspC as a source of DNA in the original ligation.

The microorganism (ATCC 39501) used in the following example was constructed by transforming an E.coli K12 prototroph with the plasmid pME98, a pAT153 derivative carrying the aspC gene on a 3.4kb fragment of DNA. All the techniques are conventional techniques readily ascertained by one of ordinary skill in the art.

The blocked amino acid of formula (II) is deprotected to yield L-3,4-dihydroxyphenylalanine. Techniques for deprotecting the blocked amino acid are well known in the art and include for example, hydrolysis using a strong acid, preferably hydrochloric or hydrobromic acid. The resulting L-3,4-dihydroxyphenylalanine may then be isolated by conventional techniques, such as, for example, pH adjustment followed by crystallization of a salt of the L-3,4-dihydroxyphenylalanine.

It has been surprising to find that in contrast to the prior art methods for preparing L-3,4-dihydroxyphenylalanine, which yield mixtures of the optical isomers, the process of the present invention yields exclusively the L-isomer of 3,4-dihydroxyphenylalanine.

The following Examples are intended to further illustrate the present invention and not to limit the invention in spirit or scope. In the Examples, all parts are parts by weight unless otherwise expressly set forth. The term "cells" as used herein in the following Examples refers to genetically modified cells containing elevated levels of the aminotransferase required to conduct the transamination.

EXAMPLE 1

E.coli containing a plasmid carrying a gene encoding for aspartate aminotransferase was grown in the following medium: glucose (35g), K $HPO_4$ (6.6g), $(NH_4)_2$ $HPO_4$ (2.0g), $MgSO_4 \cdot 7H_2O$(2.25g), ammonium ferric citrate (0.11g), indole acrylic acid (20 mg) and sufficient water to produce a final volume of 1 liter. The cells were cultivated at 33°C for 24 hours to yield a suspension having a cell density of 13.5 g. cells/l containing $4.3 \times 10^5$ U aspartate aminotransferase per liter.

EXAMPLE 2

A mixture containing 501g of vanillin (3.3 mol), 329g of hydantoin (3.29 mol) and 40g of ethanolamine (0.656 mol) in 2

liters of water was heated at 80°C for four hours to yield 3'-methoxy-4'-hydroxy-5-benzylidene hydantoin as a yellow powder which precipitates from the hot mixture. The resulting reaction mixture was cooled to room temperature and the 3'-methoxy-4'-hydroxy-5-benzylidene hydantoin precipitate was removed by filtration. The precipitate was washed with water and dried at approximately 50-60°C. A portion of the dried 3'-methoxy-4'hydroxy-5-benzylidene hydantoin (524.4g, 2.241 mol) was reacted with a solution of 896.4g sodium hydroxide (22.41 mol) in 6.5 liters of water at reflux temperature for six hours to yield a solution containing the sodium salt of 3-methoxy-4-hydroxyphenylpyruvic acid. The pH of the solution was adjusted to pH 9.5 using concentrated sulfuric acid.

To the solution containing the sodium salt of 3-methoxy-4-hydroxy phenylpyruvic acid was added 380 g of aspartic acid, 1 liter of the cell suspension prepared in Example 1, 75mg of pyridoxal phosphate and the sufficient water to yield a reaction mixture having a final volume of 10 liters. The resulting mixture was maintained at 30°C for approximately 16 hours while maintaining the pH at 8.0 using 50% $NH_4OH$ or 10% $H_2SO_4$ to yield 34.3 g of 3-methoxy-4-hydroxyphenylalanine.

The 3-methoxy-4-hydroxy phenylalanine may be hydrolyzed in the presence of a strong acid to yield L-3,4-dihydroxyphenylalanine as illustrated by the following Example.

EXAMPLE 3

A mixture containing 20mg. of L-3-methoxy-4-hydroxyphenylalanine and 5ml of a hydrobromic acid solution (48% w/v $H_2O$) was heated at a temperature of 110°C for two hours under a nitrogen atmosphere. The resultant mixture was cooled in ice to yield L-3,4-dihydroxyphenylalanine. The presence of L-3,4-dihydroxyphenylalanine was confirmed using high pressure liquid chromatography techniques.

Although this invention has been described with respect to specific modification, the details thereof are not to be construed as limitations, for it will be apparent that various equivalents, changes and modification may be resorted to without departing from the spirit and scope thereof and it is understood that such equivalent embodiments are intended to be included therein.

WHAT IS CLAIMED IS:

1. A process for preparing L-3,4-dihydroxyphenylalanine comprising:

a) reacting a blocked keto acid of the formula

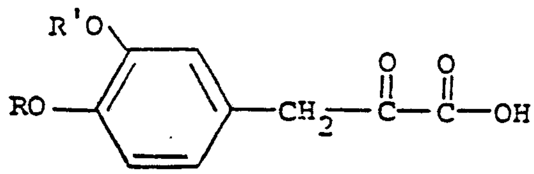

wherein R and R' are independently hydrogen or $C_1$-$C_4$ alkyl provided that R and R' are not both hydrogen; with an amine donor in the presence of an aminotransferase obtained from a microorganism which has been genetically modified to overproduce the aminotransferase, to yield a blocked amino acid of the formula

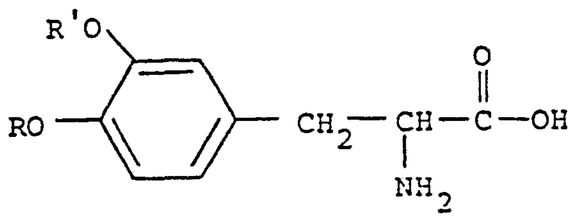

b) deprotecting the blocked amino acid to yield L-3,4-dihydroxyphenylalanine.

2. A process according to Claim 1 wherein the amine donor is glutamic acid or aspartic acid.

3. A process according to Claim 2 wherein R is hydrogen and R' is $C_1$-$C_4$ alkyl.

4. A process according to Claim 3 wherein the blocked amino acid is deprotected by reacting the blocked amino acid with a strong acid.

5. A process according to Claim 4 wherein R' is methyl.

6. A process according to Claim 3 wherein the reaction of the blocked keto acid and amine donor is conducted at a temperature equal to or greater than 40°C.

7. A process according to Claim 3 wherein the concentration of the blocked keto acid is greater than 10g/L.

8. A process according to Claim 7 wherein the reaction of the blocked keto acid and amine donor is conducted at a temperature of from about 40°C - 70°C.

9. A process according to Claim 3 wherein the amine donor is aspartic acid.

10. A process according to Claim 9 wherein the reaction of the blocked keto acid and amine donor is conducted at a temperature of from about 40°C - 70°C.

11. A process for preparing an L-3,4-dihydroxyphenylalanine comprising:

a) reacting a substituted benzaldehyde of the formula

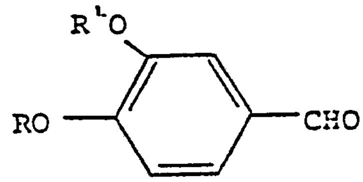

with hydantoin in the presence of an organic base to yield 3',4'-substituted-5- benzylidene hydantoins of the formula

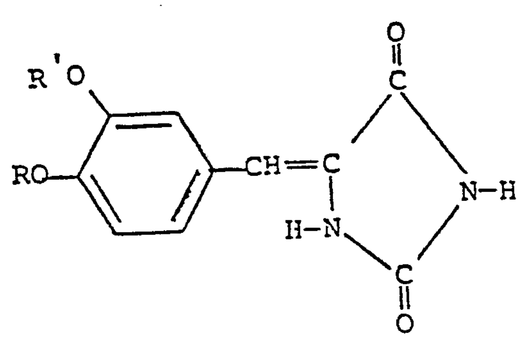

b) reacting the 3',4'-substituted-5-benzylidene hydantoin with a base to yield a blocked keto acid of the formula

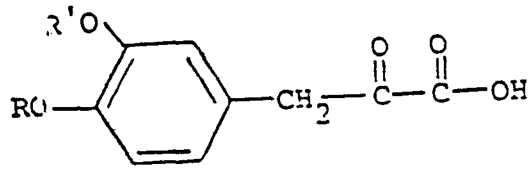

c) reacting the blocked keto acid with an amine donor in the presence of an aminotransferase obtained from a microorganism which has been genetically modified to overproduce the aminotransferase to yield a blocked amino acid of the formula

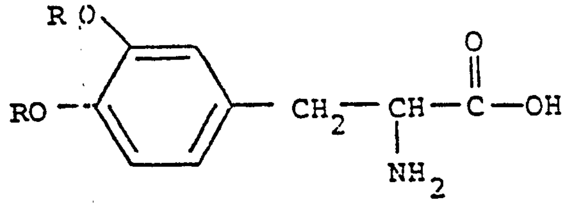

d) deprotecting the blocked amino acid to yield L-3,4-dihydroxyphenylalanine.

12. A process according to Claim 11 wherein the amine donor is glutamic acid or aspartic acid.

13. A process according to Claim 12 wherein R is hydrogen and R' is $C_1$-$C_4$ alkyl.

14. A process according to Claim 13 wherein the blocked amino acid is deprotected by reacting the blocked amino acid with a strong acid.

15. A process according to Claim 14 wherein R' is methyl.

16. A process according to Claim 13 wherein the reaction of the blocked keto acid and amine donor is conducted at a temperature greater than 40°C.

17. A process according to Claim 13 wherein the concentration of the blocked keto acid is greater than 10g/L.

18. A process according to Claim 17 wherein the reaction of the blocked keto acid and amine donor is conducted at a temperature of from about 40°C - 70°C.

19. A process according to Claim 13 wherein the amine donor is aspartic acid.

20. A process according to Claim 19 wherein the reaction of the blocked keto acid and amine donor is conducted at a temperature of from about 40°C - 70°C.